Europäisches Patentamt

European Patent Office

Office européen des brevets

(19)

(11) Veröffentlichungsnummer: **0 166 182**
**B1**

(12) **EUROPÄISCHE PATENTSCHRIFT**

(45) Veröffentlichungstag der Patentschrift:
**08.11.89**

(51) Int. Cl.⁴: **C 07 D 333/28**

(21) Anmeldenummer: **85106115.0**

(22) Anmeldetag: **18.05.85**

(54) Verfahren zur Isomerisierung halogenierter Thiophene.

(30) Priorität: **25.05.84 DE 3419555**

(43) Veröffentlichungstag der Anmeldung:
**02.01.86 Patentblatt 86/1**

(45) Bekanntmachung des Hinweises auf die Patenterteilung:
**08.11.89 Patentblatt 89/45**

(84) Benannte Vertragsstaaten:
**AT BE CH DE FR GB IT LI NL SE**

(56) Entgegenhaltungen:
**CHEMICAL ABSTRACTS, Band 95, Nr. 23, 7. Dezember 1981, Seite 574, Spalte 1, Abstract Nr. 202731m, Columbus, Ohio, US; Y. ONO: "Ring transformations of heterocycles over synthetic zeolites", & HETEROCYCLES 1981, 16(10), 1755-1771**
**CHEMICAL ABSTRACTS, Band 69, Nr. 11, 9. September 1968, Seite 4092, Spalte 2, Abstract Nr. 43711z, Columbus, Ohio, US; R.M. KELLOGG et al.: "Acid-catalyzed brominations, deuterations, rearrangements, and debrominations of thiophenes under mild conditions", & J. ORG. CHEM. 1968, 33(7), 2902-2909**
(73) Patentinhaber: **HOECHST AKTIENGESELLSCHAFT,**
**Postfach 80 03 20, D-6230 Frankfurt am Main 80 (DE)**

(72) Erfinder: **Eichler, Klaus, Dr., Im Traminer 10,**
**D-6236 Eschborn 2 (DE)**
Erfinder: **Leupold, Ernst Ingo, Dr., Am Zäunefeld 15,**
**D-6392 Neu-Anspach (DE)**

**Beschreibung**

Die vorliegende Erfindung betrifft ein Verfahren zur Isomerisierung halogenierter Thiophene.

Halogenierte Thiophene sind wertvolle Zwischenprodukte zur Herstellung von Pharmazeutika und Pflanzenschutzmitteln (Ullmanns Enzyklopädie der technischen Chemie, 4. Auflage, Band 23, Seite 219). Während sich 2-Chlorthiophen durch Chlorierung von Thiophen synthetisieren läßt, ist 3-Chlorthiophen auf diesem Wege nicht zugänglich. Zu seiner Herstellung sind daher andere Synthesewege notwendig, z.B. die Umsetzung von 3-Bromthiophen mit CuCl (S. Conde, Synthesis 1976, 6, Seite 412). 3-Bromthiophen wird durch Dehalogenierung von 2,3,5-Tribromthiophen mit Zinkstaub in Essigsäure hergestellt (Ullmanns Enzyklopädie der technischen Chemie, 4. Auflage, Band 23, Seite 219), während 2-Bromthiophen durch Bromierung von Thiophen in Essigsäure auf einfachem Wege erhalten werden kann. Alle bisher bekannten Herstellmethoden für 3-Chlor- oder 3-Bromthiophen benötigen entweder sehr teure Ausgangsstoffe oder ergeben nur unzureichende Ausbeuten. Einfache und preiswerte Methoden im technischen Maßstab wurden bisher nicht beschrieben.

Es wurde nun gefunden, daß sich halogenierte Thiophene mit guten Ausbeuten an Zeolith-Katalysatoren isomerisieren lassen.

Gegenstand der Erfindung ist daher ein Verfahren zur Isomerisierung halogenierter Thiophene, dadurch gekennzeichnet, daß man ein halogeniertes Thiophen oder ein Gemisch aus halogenierten Thiophenen mit einem Zeolith-Katalysator in Berührung bringt. Insbesondere ist Gegenstand der Erfindung ein Verfahren zur Herstellung von 3-Chlorthiophen durch Isomerisierung von 2-Chlorthiophen und ein Verfahren zur Herstellung von 3-Bromthiophen durch Isomerisierung von 2-Bromthiophen an einem Zeolith-Katalysator. Der Begriff «halogenierte Thiophene» soll alle Thiophene umfassen, die ein bis drei Halogenatome enthalten, also z.B. neben Chlor- und Bromthiophen auch Dibrom-, Tribrom-, Dichlor- und Trichlorthiophene.

Aufgrund des Standes der Technik war es überraschend und in keiner Weise vorhersehbar, daß sich halogenierte Thiophene in so einfacher Weise mit so hohen Ausbeuten, wie die Beispiele zeigen, isomerisieren lassen und daß sich bisher schwer herstellbare 3-Halogenthiophene so leicht aus technisch zugänglichen 2-Halogenthiophenen herstellen lassen.

Zur Durchführung des erfindungsgemäßen Verfahrens wird ein halogeniertes Thiophen oder ein Gemisch aus zwei oder mehr halogenierten Thiophenen entweder alleine oder zusammen mit einem oder mehreren organischen Verdünnungsmitteln mit dem Zeolith-Katalysator in Kontakt gebracht. Als organisches Verdünnungsmittel verwendet man im allgemeinen Benzol, ein Alkylbenzol, ein einfach oder mehrfach halogeniertes Benzol oder ein Gemisch aus diesen. Das Molverhältnis des Verdünnungsmittels zu dem eingesetzte Halogenthiophen oder den Halogenthiophenen beträgt im allgemeinen 0:1 bis 30:1, vorzugsweise 0:1 bis 15:1.

Als Zeolithe eignen sich im allgemeinen sowohl natürliche wie auch synthetische Zeolithe, vorzugsweise synthetische Zeolithe vom Pentasil-, Mordenit- oder Faujasit-Typ, insbesondere synthetische Zeolithe vom Pentasil-Typ.

Für den Begriff Pentasile gilt dabei die Definition von Kokotailo und Meier («Pentasil family of high silicon crystalline materials» in Special Publication No. 33 of the Chemical Society, London, 1980). Die Pentasil-Familie umfaßt beispielsweise die synthetischen Zeolithe ZSM-5 (US-A 3 702 886), ZSM-8 (GB-A 1 334 243); ZSM-11 (US-A 3 709 979) und ZSM-23 (US-A 4 076 842).

Das Si/Al-Verhältnis der Pentasile liegt vorzugsweise bei 20 bis 2000, das der Mordenite bei vorzugsweise 5 bis 100. Pentasile oder Mordenite mit einem höheren Aluminiumgehalt lassen sich dabei auf das gewünschte Si/Al-Verhältnis einstellen, indem man durch Behandlung mit Mineralsäuren, organischen Säuren oder chelatisierenden Substanzen einen Teil des Aluminiums aus dem Zeolith-Gitter entfernt.

Bei dem erfindungsgemäßen Verfahren werden die Zeolithe vorzugsweise in ihrer sauren Form eingesetzt. Diese sauren Formen lassen sich nach bekannten Methoden aus den Alkalimetall-Formen, wie sie in der Regel bei der Zeolith-Synthese anfallen bzw. als Naturprodukte vorkommen, durch vollständigen oder partiellen Ionenaustausch herstellen. Eine übliche Methode zur Herstellung der H-Form eines Zeoliths besteht beispielsweise darin, die Alkalimetall-Form zunächst durch partiellen oder vollständigen Ionenaustausch mit einer Ammoniumsalz-Lösung in die Ammoniumform und diese anschließend durch Kalzinieren in die H-Form zu überführen. Aber auch die mit Alkali-, Erdalkali- und mit Seltenerdmetall-Ionen ausgetauschten Formen zeigen katalytische Aktivität.

Die erfindungsgemäßen Zeolith-Katalysatoren bestehen im allgemeinen aus der katalytisch aktiven Zeolith-Komponente sowie einem Bindermaterial. Letzteres ist erforderlich, um den Zeolith in eine für das erfindungsgemäße Verfahren geeignete äußere Form zu bringen.

Als Bindermaterial eignen sich vor allem Oxide oder Hydroxide des Aluminiums und die Oxide bzw. Hydroxide des Siliciums, sowie Schichtsilicate, beispielsweise aus der Kaolin- oder Montmorillonit-Familie.

Dieser so hergestellte Zeolith-Katalysator wird gewöhnlich vor dem Einsatz in der erfindungsgemäßen Isomerisierungsreaktion zunächst durch Kalzinieren bei Temperaturen zwischen 300 und 700 °C aktiviert. Zur besseren Stabilisierung des Katalysators ist es manchmal vorteilhaft, die Kalzinierung in Gegenwart von Wasserdampf, Ammoniak oder deren Mischungen durchzuführen.

Falls in der Gasphase gearbeitet werden soll, besteht eine günstige einfache Verfahrensweise zur Durchführung der erfindungsgemäßen Isomerisierung darin, daß man das halogenierte Thiophen oder die halogenierten Thiophene, ggf. verdünnt wie oben erwähnt, aus einer Dosiervorrichtung zuerst in eine Verdampfungszone und das entstandene Gas dann durch ein von außen beheiztes und mit dem Katalysator gefülltes Reaktionsrohr leitet. Bei Durchführung der Isomerisierung in flüssiger Phase wird das oder die Thiophene, ggf. verdünnt, erst erwärmt und dann in flüssiger Form durch das mit dem Katalysator gefüllte Reaktionsrohr geleitet.

In der Verdampfungs- oder Erwärmungszone erfolgt gegebenenfalls noch eine Vermischung mit Wasserstoff, Stickstoff und/oder einem anderen Trägergas, wobei Wasserstoff bevorzugt wird. Es hat sich dabei als vorteilhaft erwiesen, diese Gase vor der Vermischung auf Reaktionstemperatur aufzuheizen.

Die Reaktionsprodukte werden nach Verlassen des Reaktors zur Abtrennung der kondensierbaren Anteile gekühlt. Die erfindungsgemäße Isomerisierung ist jedoch nicht auf diese Verfahrensweise (Festbett-Reaktor) beschränkt, sondern läßt sich im Prinzip auch in anderen geeigneten Reaktor-Typen durchführen (z.B. Wirbelschicht-Reaktor).

Die Belastung des Zeolith-Katalysators – ausgedrückt als LHSV (Liquid Hourly Space Velocity, $h^{-1}$) – liegt dabei im allgemeinen zwischen 0,05 und 10 $h^{-1}$, vorzugsweise zwischen 0,2 und 5 $h^{-1}$.

Die erfindungsgemäße Isomerisierung wird im allgemeinen bei Temperaturen zwischen 150 und 550 °C, vorzugsweise bei 180 bis 350 °C, sowie Drücken von 0,1 bis 10 bar, vorzugsweise bei Normaldruck, durchgeführt.

Das entstandene Isomerengemisch kann nach bekannten Verfahren destillativ getrennt werden. Das nicht umgesetzte Ausgangsprodukt kann in den Reaktor zurückgeführt werden.

Falls die Aktivität des Katalysators langsam aufgrund einer Verkokung abnimmt, kann er von Zeit zu Zeit regeneriert werden. Dies geschieht, indem Sauerstoff, Luft, Stickstoff/Luft, Sauerstoff/Luft, Sauerstoff/Inertgas oder Luft/Inertgas bei Temperaturen zwischen 300 und 650 °C über den desaktivierten Katalysator geleitet wird. Stickstoff/Luft wird dabei bevorzugt. Die Temperatur sollte dabei an keiner Stelle des Reaktors 650 °C übersteigen. Nach dem Regenerieren besitzt der Katalysator wieder die volle Aktivität, wie Beispiel 3 zeigt.

Die Erfindung soll durch die folgenden Beispiele erläutert werden, wobei diese aber in keiner Weise einschränkend sein sollen.

Beispiele
Herstellung des Katalysators

100 g ZSM-5-Pulver in der Na-Form (US-PS 3 702 886) wurden bei 100 °C dreimal für 5 Stunden mit 1 molarer Ammoniumchloridlösung behandelt, gewaschen, getrocknet und 5 Stunden bei 550 °C in Luft calciniert. 65 g des erhaltenen Pulvers wurden mit 35 g $Al_2O_3$ zu Strangpresslingen von 1,6 mm Durchmesser verarbeitet, 4 h bei 500 °C calciniert, auf eine Teilchengröße von 0,25 bis 1,0 mm zerkleinert und bei 450 °C im Stickstoffstrom zwei Stunden lang calciniert.

Beschreibung der Apparatur

15 ml des oben beschriebenen Katalysators wurden in einen Rohrreaktor aus Glas von 16 mm Innendurchmesser und 50 cm Länge eingefüllt und mit Glaskugeln (zum Verdampfen der flüssigen Reaktanden) überschichtet. Der Reaktor befand sich in einem elektrisch beheizten Ofen. Flüssige Reaktanden wurden über eine Dosierpumpe zugeführt, Gase über eine Gasversorgung, bestehend aus Reduzierventilen und Vorrichtungen zum Messen des Drucks und der Durchflußmenge. Die kondensierbaren Reaktionsprodukte wurden in einer Kühlfalle bei 0 °C kondensiert und gaschromatographisch analysiert.

Beispiel 1
(Isomerisierung von 2-Chlorthiophen)

15 ml/h eines Gemisches aus 2-Chlorthiophen und Benzol im Verhältnis 1:10 wurden mit 5 l/h Stickstoff bei einer Temperatur von 250 °C über den oben beschriebenen Katalysator geleitet. Nach einer kurzen Vorlaufzeit von 15 Minuten zur Einstellung konstanter Betriebsbedingungen wurde über einen Zeitraum von einer Stunde das Produktgemisch gesammelt und anschließend analysiert. 84,2 Gew.-% des eingesetzten 2-Chlorthiophens isomerisierten unter diesen Bedingungen zu 3-Chlorthiophen. Als Nebenprodukt wurde Thiophen gefunden (5,3 %, bezogen auf das eingesetzte 2-Chlorthiophen).

Beispiel 2
(Isomerisierung von 2-Bromthiophen)

15 ml/h eines Gemisches aus 2-Bromthiophen und Benzol im Verhältnis 1:10 wurden zusammen mit 5 l/h Stickstoff bei einer Temperatur von 210 °C über den oben beschriebenen Katalysator geleitet. Nach einer kurzen Vorlaufzeit von 15 Minuten zur Einstellung konstanter Betriebsbedingungen wurde über einen Zeitraum von vier Stunden das Produktgemisch gesammelt, indem stündlich die Vorlage gewechselt und anschließend analysiert wurde. Tabelle 1 zeigt die dabei erhaltenen Ergebnisse.

Tabelle 1
Isomerisierung von 2-Bromthiophen

| Dauer (h) | Ausbeute 3-Bromthiophen (%) | Ausbeute Thiophen (%) |
|---|---|---|
| 1 | 66,6 | 26,3 |
| 2 | 76,6 | 13,3 |
| 3 | 85,4 | 2,4 |
| 4 | 86,5 | 1,3 |

Nach einer kurzen Anfangsphase, in der relativ viel Thiophen gebildet wurde, zeigte der Katalysator sehr selektives Verhalten.

Ab der fünften Stunde wurden für zwei weitere Stunden 5 ml/h 2-Bromthiophen (ohne Benzolzusatz) und 5 l/h Stickstoff bei 210 °C über den Katalysator geleitet. Die Ausbeute an 3-Bromthiophen betrug 82,6%. Die Ausbeute des Nebenprodukts Thiophen betrug 2,8%. Weitere Nebenprodukte bildeten sich nur in Mengen unter 1%.

Beispiel 3
(Isomerisierung von 2-Bromthiophen an einem regenerierten Katalysator)

Der bereits zur Durchführung von Beispiel 2 benutzte Katalysator wurde nach insgesamt 16 Betriebsstunden mit 20 l/h Stickstoff und 5 l/h Luft eine Stunde, dann mit 20 l/h Stickstoff und 10 l/h Luft eine Stunde und anschließend noch eine Stunde mit 10 l/h Stickstoff und 10 l/h Luft bei jeweils 500 °C regeneriert.

Anschließend wurden zwei Stunden lang 5 ml/h 2-Bromthiophen und 5 l/h Stickstoff bei 210 °C über den Katalysator geleitet. Die Ausbeute an 3-Bromthiophen betrug in der ersten Stunde 74,3%, in der zweiten Stunde 79,8%, die an Thiophen in der ersten Stunde 5,8%, in der zweiten Stunde 3,5%. Dies zeigt, daß durch die Regenerierung die volle katalytische Aktivität wiederhergestellt wurde.

**Patentansprüche**

1. Verfahren zur Isomerisierung halogenierter Thiophene, dadurch gekennzeichnet, daß man ein halogeniertes Thiophen oder ein Gemisch aus halogenierten Thiophenen mit einem Zeolith-Katalysator in Berührung bringt.

2. Verfahren zur Herstellung von 3-Chlorthiophen, dadurch gekennzeichnet, daß man 2-Chlorthiophen mit einem Zeolith-Katalysator in Berührung bringt.

3. Verfahren zur Herstellung von 3-Bromthiophen, dadurch gekennzeichnet, daß man 2-Bromthiophen mit einem Zeolith-Katalysator in Berührung bringt.

4. Verfahren nach einem der Ansprüche 1–3, dadurch gekennzeichnet, daß man einen Zeolith vom Pentasil-, Mordenit- oder Faujasit-Typ einsetzt.

5. Verfahren nach einem der Ansprüche 1–3, dadurch gekennzeichnet, daß man einen sauren Zeolith vom Pentasil-, Mordenit- oder Faujasit-Typ einsetzt.

6. Verfahren nach einem der Ansprüche 1–3, dadurch gekennzeichnet, daß man einen sauren Zeolith vom Pentasil-Typ einsetzt.

7. Verfahren nach Anspruch 5 oder 6, dadurch gekennzeichnet, daß der saure Zeolith Protonen als Kationen enthält.

8. Verfahren nach einem der Ansprüche 1 bis 7, dadurch gekennzeichnet, daß man in Gegenwart von Benzol, Alkylbenzolen oder halogenierten Benzolen oder einem Gemisch aus diesen arbeitet.

9. Verfahren nach einem der Ansprüche 1 bis 8, dadurch gekennzeichnet, daß man bei Temperaturen zwischen 150 und 450 °C und bei einem Druck zwischen 0,1 und 10 bar arbeitet.

10. Verfahren nach einem der Ansprüche 1 bis 9, dadurch gekennzeichnet, daß man in Gegenwart von Wasserstoff, Stickstoff, Wasserdampf, Argon oder einem Gemisch aus diesen arbeitet.

**Claims**

1. A process for the isomerization of halogenated thiophenes, which comprises bringing a halogenated thiophene or a mixture of halogenated thiophenes into contact with a zeolite catalyst.

2. A process for the preparation of 3-chlorothiophene, which comprises bringing 2-chlorothiophene into contact with a zeolite catalyst.

3. A process for the preparation of 3-bromothiophene, which comprises bringing 2-bromothiophene into contact with a zeolite catalyst.

4. The process as claimed in any one of claims 1–3, wherein a zeolite of the pentasil, mordenite or faujasite type is employed.

5. The process as claimed in any one of claims 1–3, wherein an acid zeolite of the pentasil, mordenite or faujasite type is employed.

6. The process as claimed in any one of claims 1–3, wherein an acid zeolite of the pentasil type is employed.

7. The process as claimed in either of claims 5 and 6, wherein the acid zeolite contains protons as cations.

8. The process as claimed in any one of claims 1 to 7, wherein the reaction is carried out in the presence of benzene, an alyklbenzene or a halogenated benzene or a mixture of these.

9. The process as claimed in any one of claims 1 to 8, wherein the reaction is carried out at a temperature between 150 and 450 °C under a pressure of between 0.1 and 10 bar.

10. The process as claimed in any one of claims 1 to 9, wherein the reaction is carried out in the presence of hydrogen, nitrogen, steam, argon or a mixture of these.

**Revendications**

1. Procédé d'isomérisation de thiophènes halogénés, caractérisé en ce qu'on met en contact, avec un catalyseur à base de zéolite un thiophène halogéné ou un mélange de thiophènes halogénés.

2. Procédé de préparation du chloro-3 thiophène, caractérisé en ce qu'on met en contact du chloro-2 thiophène avec un catalyseur à base de zéolite.

3. Procédé de préparation du bromo-3 thiophène, caractérisé en ce qu'on met en contact du bromo-2 thiophène avec un catalyseur à base de zéolite.

4. Procédé selon l'une des revendications 1 à 3, caractérisé en ce qu'on utilise une zéolite du type pentasil, mordénite ou faujasite.

5. Procédé selon l'une des revendications 1 à 3, caractérisé en ce que l'on utilise une zéolite acide du type pentasil, mordénite ou faujasite.

6. Procédé selon l'une des revendications 1 à 3, caractérisé en ce qu'on utilise une zéolite acide du type pentasil.

7. Procédé selon la revendication 5 ou 6, caractérisé en ce que la zéolite acide contient des protons comme cations.

8. Procédé selon l'une des revendications 1 à 7, caractérisé en ce qu'on travaille en présence de benzène, d'alkylbenzènes ou de benzènes halogénés ou d'un de leurs mélanges.

9. Procédé selon l'une des revendications 1 à 8, caractérisé en ce qu'on travaille à des températures comprises entre 150 et 450°C et sous une pression comprise entre 0,1 et 10 bars.

10. Procédé selon l'une des revendications 1 à 9, caractérisé en ce qu'on travaille en présence d'hydrogène, d'azote, de vapeur d'eau, d'argon ou d'un de leurs mélanges.